# EUROPÄISCHE PATENTSCHRIFT

(11) **EP 1 528 934 B1**
(45) Veröffentlichungstag und Bekanntmachung des Hinweises auf die Patenterteilung: **07.01.2015**
(21) Anmeldenummer: 03783820.8
(22) Anmeldetag: 31.07.2003
(51) Int. Cl.: A61K 39/395, A61P 35/00, C07K 16/30

(54) **VERWENDUNG VON ANTIKÖRPERN GEGEN EIN TUMOR-ASSOZIIERTES ANTIGEN**
USE OF ANTIBODIES AGAINST A TUMOR-ASSOCIATED ANTIGEN
UTILISATION D'ANTICORPS AGISSANT A L'ENCONTRE D'UN ANTIGENE TUMORAL

(30) Priorität: 12.08.2002 AT 12172002
(43) Veröffentlichungstag der Anmeldung: 11.05.2005
(73) Patentinhaber: greenovation Biotech GmbH, 79111 Freiburg (DE); Meridian Biopharmaceuticals GmbH, 1230 Wien (AT)
(72) Erfinder: LOIBNER, Hans, 1238 Wien (AT); HIMMLER, Gottfried, 1180 Wien (AT); SAMONIGG, Hellmut, 8043 Graz (AT); STERMETZ, Eugen, 1010 Wien (AT); HAUER, Andreas, 1160 Wien (AT); REDL, Gerda, 2301 Rutzendorf (AT)
(74) Vertreter: Sonn & Partner Patentanwälte
(86) Internationale Anmeldenummer: PCT/AT2003/000219
(87) Internationale Veröffentlichungsnummer: WO 2004/014421

(56) Entgegenhaltungen:
- WO-A-00/69460
- NAKASHIO T ET AL: "PERITONEAL DISSEMINATION IS INHIBITED BY TREATMENT WITH ANTIBODIES AGAINST CD44H, BETA1 INTEGRIN, AND CARCINOSTATIC AGENTS IN NUGC-4 HUMAN GASTRIC CANCER CELLS" INTERNATIONAL JOURNAL OF ONCOLOGY, EDITORIAL ACADEMY OF THE INTERNATIONAL JOURNAL OF ONCOLOGY,, GR, Bd. 10, Nr. 2, 1997, Seiten 355-362, XP008024710 ISSN: 1019-6439
- SCHNEIDER-GAEDICKE E ET AL: "Prevention of Manifest Metastasis with Monoclonal Antibodies: A Novel Approach to Immunotherapy of Solid Tumours" EUROPEAN JOURNAL OF CANCER, Bd. 31A, Nr. 7-8, 1995, Seiten 1326-1330, XP002263000 ISSN: 0959-8049
- RIETHMUELLER G ET AL: "Monoclonal antibody therapy for resected Dukes' C colorectal cancer: Seven-year outcome of a multicenter randomized trial" JOURNAL OF CLINICAL ONCOLOGY, PHILADELPHIA, PA, US, Bd. 16, Nr. 5, Mai 1998 (1998-05), Seiten 1788-1794, XP009016638
- RAMSLAND P A ET AL: "Structural Convergence of Antibody Binding of Carbohydrate Determinants in Lewis Y Tumor Antigens", JOURNAL OF MOLECULAR BIOLOGY, LONDON, GB, vol. 340, no. 4, 16 July 2004 (2004-07-16) , pages 809-818, XP004516692, ISSN: 0022-2836
- POWER BARBARA E ET AL: "Construction, expression and characterisation of a single-chain diabody derived from a humanised anti-Lewis Y cancer targeting antibody using a heat-inducible bacterial secretion vector", CANCER IMMUNOLOGY IMMUNOTHERAPY, vol. 50, no. 5, July 2001 (2001-07), pages 241-250, ISSN: 0340-7004
- CO MAN SUNG ET AL: "Humanized anti-Lewis Y antibodies: In vitro properties and pharmacokinetics in rhesus monkeys", CANCER RESEARCH, vol. 56, no. 5, 1996, pages 1118-1125, ISSN: 0008-5472

## Beschreibung

Die Erfindung betrifft eine neue Verwendung eines Antikörperpräparates sowie ein Set zur intraoperativen Behandlung von Tumorpatienten.

Tumore entstehen aufgrund von unkontrolliertem Zellwachstum, was bei epithelialen Zellen zur Ausbildung von soliden Zellagglomeraten führt. Im Fall von gutartigen Tumorgewebe geht man davon aus, dass das Zellwachstum limitiert ist und keine Sekundärtumore oder Metastasen auftreten. Bei Krebserkrankungen kommt es jedoch zur Bildung von malignen Tumoren, wobei im fortlaufenden Stadium Sekundärtumoren und Metastasen vorkommen. Am häufigsten treten Krebsformen mit epithelialen Tumoren auf, die unter anderem Brust, Magen, Darm, Pankreas, Lunge, Prostata und Eierstock betreffen.

Krebs ist eine weit verbreitete Krankheit und verläuft in vielen Fällen tödlich. Die Krebstherapie umfasst üblicherweise die Entfernung eines soliden Tumors und eine weitere Behandlung, die Metastasen verhindern bzw. reduzieren soll. Zu den Standardtherapien zählen neben der Chirurgie noch die Chemotherapie und Bestrahlungstherapie. Trotz der umfassenden Therapie, die oft mit schweren Nebenwirkungen einher geht, ist der Behandlungserfolg ungenügend. Die Rückfallsrate bei Darmkrebs liegt bei etwa 45%. Metastatischer epithelialer Krebs gilt nahezu als unheilbar. Es gilt daher bei der Behandlung von Krebspatienten die Metastasenbildung zu verhindern bzw. zu reduzieren.

Tumorzellen können von Primärtumoren in Körperflüssigkeiten und anderen Organen disseminieren. Diese disseminierten Tumorzellen können sich im Ruhezustand befinden und sind durch eine Chemotherapie (Radiotherapie) oft nicht angreifbar. Ein derartig behandelter Patient befindet sich scheinbar in einem geheilten Zustand, welcher auch als "minimal residual disease" beschrieben ist. Ruhende Tumorzellen haben jedoch ein Potential zur Metastasenbildung, wenn sie zu wachsenden und metastasierenden Zellen werden.

Die Immuntherapie stellt eine innovative Behandlungsmöglichkeit von Krebspatienten dar. Sowohl aktive als auch passive Immuntherapie sind anerkannte Maßnahmen zur Unterstützung des Immunsystems.

Das adaptive Immunsystem des Menschen besteht aus zwei wesentlichen Komponenten, der humoralen und der zellulären Immunität. Die adaptive Immunantwort beruht zum Teil auf der klonalen Selektion von B- und T-Lymphozyten und erlaubt im Prinzip die Erkennung jedes beliebigen Antigens sowie den Aufbau eines immunologischen Gedächtnisses. Diese Merkmale des adaptiven Immunsystems werden generell bei Impfungen nutzbringend angesprochen.

Jede B-Zelle produziert einen Antikörper mit bestimmter Bindungsspezifität. Dieser Antikörper befindet sich als spezifischer Rezeptor auch in der Membran der ihn produzierenden B-Zelle. Die humorale Immunantwort gegen als fremd erkannte Antigene beruht auf der selektiven Aktivierung derjenigen B-Zellen, die solche Antikörper produzieren, die an Epitope des jeweiligen Antigens binden können. Für die Antikörpervielfalt spielen DNA-Rearrangements im Verlauf der B-Zelldifferenzierung eine entscheidende Rolle.

Es gibt mehrere Möglichkeiten, in das Immunsystem einzugreifen.

### 1. Passive Antikörpertherapie:

Für therapeutische Zwecke ist es möglich, Antikörper, die notwendig sind, um eine bestimmte Funktion innerhalb eines Organismus zu erfüllen, diesem Organismus zuzuführen. Diese Art der Anwendung wird passive Immuntherapie genannt und kann bei verschiedenen medizinischen Indikationen eingesetzt werden, z.B. bei Krebs-Immuntherapie (Immunol. Today (2000), 21:403), Vergiftungen (Toxicon (1998), 36:823; Therapie (1994), 49:41), Infektionen (Clin. Infect. Dis. (1995), 21:150). In diesen Fällen können Antikörper eingesetzt werden, die entweder aus entsprechend immunisierten Tieren stammen oder über Immortalisierung von Immunglobulin-Genen durch verschiedene biologische oder molekularbiologische Techniken aus Zellen gewonnen werden können (z.B. Hybridoma-Technik, Phage-Display-Technik, etc.).

### 2. Aktive Immunisierung:

Zur Modulation des Immunsystems kann man mit Antigenen immunisieren. Antigene sind Moleküle, Molekül-Komplexe oder ganze Organismen, an welche Antikörper binden können. Nicht alle Antigene rufen eine Immunantwort hervor, d.h. nicht alle Antigene sind immunogen. Bestimmte kleine Moleküle werden vom Immunsystem nicht registriert (Haptene), solche kleinere Moleküle kann man dem Immunsystem in geeigneter Form präsentieren und sie dadurch immunogen machen. Eine derartige Methode ist die Kopplung des Haptens an ein immunogenes Molekül, ein sogenanntes "Trägermolekül". Zur aktiven Immunisierung können auch Antikörper-Präparate eingesetzt werden, wie in der EP 1140168 beschrieben.

Tumorzellen können durch das Immunsystem nur bedingt angegriffen werden, da sie sich von normalen Zellen kaum unterscheiden und daher spezifische Antikörper fehlen. Viele Arbeiten beschäftigen sich mit der Identifizierung geeigneter Targets, also Zielantigene zur Herstellung von Tumor-spezifischen Antikörpern. Die Immuntherapie zur Behandlung von Krebs umfasst dann entweder die passive Therapie durch die direkte Verabreichung der spezifischen Antikörper oder die aktive Impfung mit geeigneten Antigen-Targets zur Stimulierung des Immunsystems und Generierung der spezifischen Antikörper in vivo.

Ein Ansatz, Tumorzellen relativ spezifisch zu zerstören, ist die passive Immuntherapie mit Antikörpern, die gegen Tumor-assoziierte Antigene (TAA) gerichtet sind (Immunology Today (2000), 21:403-410; Curr. Opin. Immunol. (1997), 9:717). Ein anderer Ansatz Tumorzellen zu zerstören ist eine aktive Impfung, die eine Immunantwort gegen TAA auslöst. Diese Immunantwort ist somit auch gegen die entsprechenden Tumorzellen gerichtet (Ann. Med. (1999), 31:66; Immunobiol. (1999), 201:1).

Bestimmte TAA werden als relevante "targets zur Entwicklung von immuntherapeutischen Mitteln zur Prophylaxe und/oder Behandlung von Krebs definiert. TAA sind Strukturen, die bevorzugt auf der Zellmembran von Tumorzellen exprimiert sind, dadurch eine Unterscheidung zu nicht-malignem Gewebe ermöglichen und daher als Zielpunkte für diagnostische und therapeutische Anwendungen von spezifischen Antikörpern gesehen werden.

Im Verlauf der Entdeckung und nachfolgenden Charakterisierung von verschiedenen TAA hat sich herausgestellt, dass diese oft wichtige Funktionen für Krebszellen haben. Sie ermöglichen den entarteten Zellen, charakteristische Eigenschaften für den malignen Phänotyp wie z.B. vermehrte Adhäsionsfähigkeit oder vermehrte Aufnahme von Wachstumsfaktoren, auszuüben, die für die Etablierung von Metastasen von großer Bedeutung sind. Allerdings können solche Antigene durchaus in bestimmten Stadien auch auf normalen Zellen exprimiert sein, wo sie für normale Funktionen dieser Zellen verantwortlich sind. Ein Beispiel dafür ist das Lewis Y Kohlenhydratantigen, das auf der Mehrzahl der Tumoren epithelialen Ursprungs aufscheint, aber auch während der fötalen Entwicklung epithelialer Gewebe eine wichtige Rolle spielt. Es wurde gezeigt, dass die Expression dieses Antigens in Lungenkrebs mit einer ungünstigen Prognose assoziiert ist, da Lewis Y positive Krebszellen offensichtlich ein höheres metastatisches Potential haben (N. Engl. J. Med. 327 (1992), 14).

In der EP 0 528 767 ist die Verwendung eines humanisierten anti-Lewis Y-Antikörpers zur Behandlung von epithelialem Krebs beschrieben.

Unter den weiter bekannten Tumor-assoziierten Kohlenhydratstrukturen finden sich z.B. alle Lewis-Antigene, die verstärkt in vielen epithelialen Krebsarten exprimiert werden. Dazu gehören Lewis x-, Lewis b- und Lewis y-Strukturen, sowie sialylierte Lewis x-Strukturen. Andere Kohlenhydrat-Antigene sind GloboH-Strukturen, KH1, Tn-Antigen, TF-Antigen, das alpha-1,3-Galactosyl-Epitop (Elektrophoresis (1999), 20:362; Curr. Pharmaceutical Design (2000), 6:485, Neoplasma (1996), 43:285).

Andere TAA sind Proteine, die von Krebszellen besonders stark exprimiert werden, wie z.B. CEA, TAG-72, MUC1, Folate Binding Protein A-33, CA125, EpCAM, HER-2/neu, PSA, MART, etc. (Sem. Cancer Biol. (1995), 6:321). Relevante TAA sind oftmals Oberflächenantigene von epithelialen Zellen, die vermehrt in wachsenden Zellen, wie fötalem Gewebe, und auch an Tumorgewebe auftreten.

Direkte therapeutische Anwendungen von Antikörpern gegen TAA beruhen auf passiven Immuntherapien, das heißt, ein spezifischer Antikörper wird systemisch in geeigneter Menge an Krebspatienten verabreicht und übt eine immuntherapeutische Wirkung aus. Die biologische Halbwertszeit solcher Agentien hängt von ihrer Struktur ab und ist begrenzt. Daher ist es notwendig, wiederholte Applikationen vorzunehmen. Das kann bei Verwendung von xenogenen Antikörpern (z.B. murine monoklonale Antikörper, MAK) aber zu unerwünschten Immunreaktionen führen, die eine mögliche therapeutische Wirkung neutralisieren und gefährliche Nebenwirkungen (anaphylaktische Reaktionen) bedingen können. Daher können solche Immuntherapeutika nur für eine begrenzte Zeit verabreicht werden.

Eine bessere Verträglichkeit wird durch Reduktion der xenogenen Strukturen des Antikörpers und den Einbau von menschlichen Strukturen erzielt, beispielsweise mit chimären oder humanisierten Antikörpern. Es werden auch Systeme zur Herstellung spezifischer humaner Antikörper entwickelt. So können bestimmte Zelllinien, Organismen oder transgene Tiere gemäß Stand der Technik humane Antikörper produzieren.

Verschiedene Arbeiten behandeln das Risiko der Metastasenbildung durch die Freisetzung von Tumorzellen, wie sie bei einer chirurgischen Behandlung vorkommen kann. Wie in Clin. Cancer Res. 4(2), 343-8 (1998) beschrieben, können gerade während einer Tumorresektion Tumorzellen in Blutproben bestimmt werden. Die intraoperative Disseminierung hämatogener Tumorzellen wird unmittelbar mit der "minimal residual disease" sowie der Metastasenbildung in Zusammenhang gebracht. In Semin. Surg. Oncol. 20(4), 329-33 (2001) wird daher vorgeschlagen, zusätzliche peri-operative Maßnahmen der Antikörpertherapie oder zytotoxischen Therapie zu setzen.

Eine kombinierte Behandlung von Tumorpatienten wird in der WO 00/69460 beschrieben. Patienten werden zunächst 8-24 Wochen lang vor einer chirurgischen Behandlung mit einer Antikörper-Präparation, kombiniert mit einem zytotoxischen Mittel, in wöchentlichen Abständen behandelt, um die Tumorgröße zu reduzieren. Nach erfolgter Tumorresektion wird der Patient abermals der kombinierten immuntherapeutischen Behandlung unterzogen. Diese perioperative Behandlung umfasst vorerst einen vollständigen Behandlungszyklus Wochen vor und später einen weiteren Zyklus Wochen nach der Operation. In diesem Dokument werden Antikörper verabreicht, die über eine ADCC- bzw. CDC-Funktion zum Zelltod führen (s. Seite 5, Zeilen 10-17). Die so behandelten Patienten zeigen eine verbesserte Überlebensrate sowie eine verringerte Tumorausbreitung (TTP).

Gemäß der US 20010006618 werden Antikörperpräparationen für die diagnostische Verwendung während eines operativen, intravaskulären, laparoskopischen oder endoskopischen Eingriffs vorgeschlagen. Intraoperative Läsionen sollen unter Verwendung einer markierten Antikörper-Präparation detektiert werden. Dabei wird der markierte Antikörper innerhalb von 48 Stunden vor dem Eingriff verabreicht und nach der Operation der Patient hinsichtlich der möglichen Verteilung des markierten Antikörpers untersucht. Die Markierungssubstanz kann weiters außer zur Detektion auch zur Behandlung des Tumors verwendet werden, etwa indem ein fotoaktives Reagens als Markierungssubstanz eingesetzt wird, das nach Bindung des Antikörpers durch Lichteinwirkung aktiviert wird.

Gemäß der US 4643971 wird eine Auswahl von Tumor-spezifischen Antikörpern zur Bestimmung von Gallenkrebs vor einer entsprechenden Operation vorgeschlagen.

WO 01/23005 betrifft die Verabreichung von Antikörpern, die mit einem Farbstoff konjugiert sind, zur Tumorranddarstellung. Die Antikörperfarbstoffkonjugate reichern sich im Randbereich des Zellgewebes eines Tumors bevorzugt an und machen damit den Randbereich optisch darstellbar (s. Seite 8, 1. vollständiger Absatz).

Nakashio et al., Int. J. Onc. 10(2), 1997: 355-362, betrifft die Verabreichung von Antikörpern gegen die TAAs CD44H und β₁-Integrin nach einer Operation zur Entfernung eines Tumors, um eine Ausbreitung des Krebses zu verhindern oder zu verringern.

Die Erfindung stellt sich zur Aufgabe, die Behandlung von Krebspatienten insofern zu verbessern, dass die Disseminierung von Tumorzellen bzw. die Metastasenbildung weitgehend unterdrückt wird.

Die Aufgabe wird erfindungsgemäß durch den Gegenstand der Ansprüche gelöst.

Die erfindungsgemäße Verwendung betrifft ein Präparat auf Basis eines Antikörpers für die Herstellung eines Arzneimittels, welcher Antikörper gegen Lewis Y gerichtet ist. Dieses Arzneimittel wird zur intraoperativen Behandlung von Tumorpatienten im Rahmen von chirurgischen Eingriffen verabreicht, wobei epitheliale Tumorzellen immunkomplexiert werden. Die Immunkomplexbildung erfolgt während der Operation, also vor allem auf Tumorgewebe oder Zellen, die intraoperativ zugänglich werden. Zur intraoperativen Behandlung wird das Arzneimittel unmittelbar während oder innerhalb von 24 Stunden vor dem chirurgischen Eingriff verabreicht.

Die intraoperative Behandlung ist Voraussetzung für die funktionelle Aktivierung des Immunsystems gegen die Tumorzelle, worauf diese unmittelbar lysiert wird. Die Disseminierung von Tumorzellen, die während bestimmter chirurgischer Vorgänge passieren kann, wird dann weitgehend unterbunden. Dass eine Disseminierung von Tumorzellen sogar während der Biopsie, also eines relativ gering invasiven chirurgischen Eingriffs auftreten kann, wurde beispielsweise an Brustkrebs-Patientinnen gezeigt. Hier war ein Nachweis von disseminierten Tumorzellen vor der Operation nicht möglich, nach der Biopsie stieg der Wert von zirkulierenden Epithelialzellen, die disseminierten Tumorzellen entsprechen, in einigen Fällen stark an.

Das Arzneimittel wird vor allem erfindungsgemäß prophylaktisch eingesetzt, um die Disseminierung der Tumorzellen in Sekundärorgane oder in Knochenmark zu verhindern.

Die erfindungsgemäße Behandlung umfasst sowohl prophylaktische als auch therapeutische Maßnahmen. Die Behandlung dient nicht nur der Humanmedizin, sondern kann auch bei chirurgischen Eingriffen an verschiedenen Säugetieren indiziert sein.

Unter dem Begriff "intraoperative Behandlung" wird erfindungsgemäß die Behandlung von Tumorpatienten verstanden, die einen hohen Antikörpertiter während der Dauer einer Operation gewährleistet. Praktischerweise wird das Antikörper-Präparat nur wenige Stunden vorher, also unmittelbar während der Vorbereitung des Patienten für die Operation oder während der Operation selbst verabreicht. Die Behandlung wird innerhalb von 24 Stunden vor der Operation vorgenommen, vorzugsweise innerhalb von 8 Stunden, weiter bevorzugt innerhalb von 4 Stunden, am meisten bevorzugt innerhalb von einer Stunde vor dem chirurgischen Eingriff vorgenommen. Die erfindungsgemäße intraoperative Behandlung unterscheidet sich somit von einer perioperativen Behandlung des Standes der Technik, welche eine mehrwöchige und wiederholte Behandlung des Patienten vor der Operation, sowie eine weitere Behandlungsperiode nach der Operation vorsieht.

Erfindungsgemäß werden chirurgischen Eingriffe zur teilweisen oder gänzlichen Tumorresektion solider Tumoren vorgenommen. Tumorgewebe wird vorzugsweise mit einer "no touch" Operationstechnik entfernt, um die Gefahr der Disseminierung von Tumorzellen in die Peripherie von vornherein zu reduzieren.

Unmittelbar vor bzw. während des operativen Eingriffs erhält der Tumorpatient erfindungsgemäß üblicherweise eine einmalige Dosis eines Antikörper-Präparates, um einen bestimmten Serumgehalt zu erreichen. Ab einem spezifischen Immunglobulingehalt von etwa mindestens 10, vorzugsweise mindestens 50, am meisten bevorzugt mehr als 100 µg/ml Serum, wird so ein Schutz vor einer möglichen Verteilung von Tumorzellen in die Blutbahn aufgebaut. Der Antikörpertiter ist nach einer intravenösen Behandlung praktisch sofort verfügbar. Weitere systemische Behandlungen umfassen die intramuskuläre, subkutane, intradermale oder mukosale, wie orale oder nasale Verabreichung. Dabei muss aber mit einer verzögerten Verfügbarkeit gerechnet werden. Das Antikörper-Präparat kann auch lokal, also zum Tumorgewebe und/oder in den Wundbereich nach Entfernung des Tumors, verabreicht werden. Verschiedene Verabreichungsarten können auch sinnvollerweise kombiniert werden, etwa eine i.v. Infusion kurz vor der Operation, sowie eine lokale Dosis direkt in den Wundbereich appliziert. Praktische Applikationsformen für die lokalisierte Verabreichung umfassen beispielsweise fertige "ready to use" Applikatoren, etwa Katheter, Spritzen oder Sprays, geeignet zur Verteilung von Flüssigarzneimitteln.

Gerade bei endoskopischen oder mikroinvasiven Operationsmethoden ist die lokale Applikation vorteilhaft. So wird etwa bei der Biopsie das Arzneimittel vorzugsweise über den Stichkanal oder parallel über entsprechende Spritzen bzw. Katheter eingebracht.

Kleine Tumoren oder eine Probe von Tumorgewebe wird üblicherweise durch Biopsie entnommen. Aufgrund des Biopsiematerials diagnostiziert dann ein Pathologe, ob das Gewebe von einem gutartigen oder bösartigen Tumor stammt. Patienten, die biopsiert werden, werden an sich noch keiner Krebstherapie unterzogen. Der Patient erhält gemäß Stand der Technik erst nach dem positiven Befund einer Krebserkrankung entsprechende Arzneimittel. Erfindungsgemäß erhält hingegen bereits ein Risikopatient die entsprechende prophylaktische Behandlung, um eine Disseminierung von Tumorzellen aufgrund der Biopsie selbst auszuschließen.

Das erfindungsgemäß verwendete Arzneimittel ist an sich gut verträglich und wird dem Tumorpatienten üblicherweise nur einmalig verabreicht. Die Prophylaxe ist daher auch dann angebracht, wenn sich das Risiko einer Krebserkrankung noch nicht bestätigt hat, und der chirurgische Eingriff zur Bestimmung über die Malignität eines Tumors vorgenommen wird. Neben der üblichen Diagnostik durch den pathologischen Befund besteht erfindungsgemäß weiter die Möglichkeit, die entstandenen Immunkomplexe zu bestimmen und dadurch einen Indikator für die Malignität des Tumors zu erhalten. Dies setzt voraus, dass der verwendete Antikörper gegen ein TAA gerichtet ist, der gutartiges von bösartigem Tumorgewebe unterscheidet.

Im Fall einer Krebserkrankung werden erfindungsgemäß Immunkomplexe mit Tumorgewebe bzw. Tumorzellen erhalten, die nicht nur am Tumorgewebe nach Tumorresektion bestimmbar sind, sondern auch in peripheren Körperflüssigkeiten. So können Blut- oder Serumproben des Patienten nach der Behandlung qualitativ und/oder quantitativ auf Immunkomplexe untersucht werden. Die Analysenmethoden sind an sich übliche Analysenmethoden mit Fraktionierung und Anreicherung der Immunkomplexe und/oder Immunreaktion mit einem spezifischen Antikörper, etwa gerichtet gegen den Fc-Teil des zur Behandlung eingesetzten Antikörpers. Wenn der verwendete Antikörper murine Strukturen aufweist, kann dieser auch mit einem anti-murin Antikörper gebunden werden, im Sinne eines "capture" Schrittes. Der Nachweis der Bindung erfolgt üblicherweise mit einer entsprechenden Antikörper-Markierung, etwa mit einem Fluoreszenz-Label oder einer Farbreaktion.

Beschrieben wird weiter ein Set zur intraoperativen Behandlung von Tumorpatienten, enthaltend
a)ein Arzneimittel auf Basis eines Antikörpers, gerichtet gegen ein Tumor-assoziiertes Antigen, und
b)ein Mittel zur diagnostischen Bestimmung von malignen Tumorzellen, die mit dem Antikörper immunkomplexiert sind.

Der Antikörper des Sets ist dabei vorzugsweise ein nativer, also ein funktionell aktiver Antikörper. Dieser Antikörper ist daher vorzugsweise nicht mit einem Label oder anderen Detektionsmitteln behaftet, um die Funktionalität nicht zu beeinträchtigen.

Die erfindungsgemäße Verwendung wird mit Antikörpern vorgenommen, die gegen Lewis Y gerichtet sind. Gegebenenfalls wird ein TAA von Zellen solider Tumoren oder eine Selektion ("panel") von TAA gewählt, insbesondere individuelle TAA des Tumors des jeweiligen Patienten. Patienten mit Primärtumoren können gleichermaßen behandelt werden, wie Patienten mit Sekundärtumoren.

In der Regel ist davon auszugehen, dass unter einem Antigen, das ein proteinäres Epitop eines TAA imitiert, ein Polypeptid von mindestens fünf Aminosäuren zu verstehen ist.

Unter weiteren beschriebenen Epitopen finden sich Epitope eines humanen Antigens ausgesucht aus der Gruppe der Peptide oder Proteine, insbesondere EpCAM, NCAM, CEA und T-Zell Peptide, welche vorzugsweise von Tumor-assoziierten Antigenen abgeleitet sind, weiter der Kohlenhydrate, insbesondere Lewis Y, Sialyl-Tn, Globo H, und der Glycolipide, insbesondere GD2, GD3 und GM2. Bevorzugte Epitope sind von Antigenen abgeleitet, die spezifisch für epitheliale Tumoren sind und etwa vermehrt bei Brustkrebs, Krebs des Magen und Darms, der Prostata, Pankreas, Ovarien und der Lunge vorkommen. Unter den bevorzugten Epitopen sind diejenigen zu finden, welche vor allem eine humorale Immunantwort, also eine spezifische Antikörperbildung in vivo hervorrufen. Der erfindungsgemäße immunogene Antikörper kann vorzugsweise auch eine T-Zell spezifische Immunantwort auslösen, wodurch als Reaktion auf die Verabreichung des Antikörpers nicht nur Antikörper beispielsweise der IgM-Klasse gebildet werden, sondern auch der Ig-G-Klasse.

Alternativ können speziell auch diejenigen Antigene als Epitope ausgewählt werden, welche eine T-Zell spezifische Immunantwort generieren. Darunter sind vor allem auch intrazelluläre Strukturen bzw. T-Zell Peptide zu finden. Beispielsweise kann eine weiters beschriebene intraoperative Behandlung auch dazu verwendet werden, um das Volumen eines Pleuraergusses, d.h. einer Flüssigkeitsansammlung in der Pleurahöhle, zu reduzieren. Pleuraergüsse kommen häufig bei Patienten vor, die an epithelialem Krebs erkrankt sind.

Weitere beschriebene proteinäre Epitope, die besonders auf Krebszellen solider Tumoren exprimiert werden, sind z.B. TAG-72, MUC1, Folate Binding Protein A-33, CA125, HER-2/neu, EGF-Rezeptoren, PSA, MART etc. (s. z.B. Sem. Cancer Biol. 6 (1995), 321). Weiters können auch sogenannte T-Zell-Epitop-Peptide (Cancer Metastasis Rev. 18 (1999), 143; Curr. Opin. Biotechnol. 8 (1997), 442; Curr. Opin. Immunol. 8 (1996), 651) dienen.

Weitere beschriebene Epitope werden zumindest in 20%, vorzugsweise mindestens in 30% der Fälle von Tumorzellen einer bestimmten Krebsart exprimiert, weiter bevorzugt in zumindest 40%, insbesondere in zumindest 50% der Patienten.

Weitere beschriebene Kohlenhydrat-Epitope sind Tumor-assoziierte Kohlenhydratstrukturen, wie die Lewis-Antigene, z.B. Lewis x-, Lewis b- und Lewis y-Strukturen, sowie sialylierte Lewis x-Strukturen. Weiters sind auch GloboH-Strukturen, KH1, Tn-Antigen, TF-Antigen, das alpha-1-3,Galactosyl-Epitop bekannte Kohlenhydrat-Antigen-Strukturen.

Das Target für den erfindungsgemäß eingesetzten Antikörper ist das Lewis Y-Antigen. Besonders bevorzugt ist ein humanisierter Antikörper, wie in der EP 0 528 767 beschrieben. Dieser Antikörper erkennt Lewis Y-Antigen an Tumorzellen bzw. Oberflächenrezeptoren von Tumorzellen.

Es hat sich herausgestellt, dass die Oberflächenrezeptoren einer Tumorzelle mit einer aberranten Glykosilierung relevante Epitope im Sinne der Erfindung ausbilden, wodurch diese Glykosilierung durch Antikörper funktionell blockiert werden kann. Dies betrifft nicht nur einen bestimmten Oberflächenrezeptor, wie den EGF-Rezeptor oder Her-2/neu Rezeptor. Es werden gleichsam alle Tumor - spezifischen Rezeptoren, die durch die aberrante Glykosilierung charakterisiert sind, gleichzeitig blockiert. Darunter sind etwa alle Rezeptoren der EGF-Rezeptorfamilie, der CD55 (791Tgp72/DAF - decay accelerating factor) - Rezeptor, der Transferrinrezeptor und das P-Glycoprotein. Die Tumorzelle wird damit aufgrund von verschiedenen Wirkmechanismen angegriffen.

Es hat sich auch herausgestellt, dass Antikörper, die gegen eine aberrante Glykosilierung gerichtet sind, in funktioneller Weise an mehrere Rezeptoren der Familie der EGF-Rezeptoren binden und damit die Signalkaskade zur Induktion des Zellwachstums effektiv blockiert werden kann. Es konnte in der österreichischen Anmeldung A 995/2002, Publikationsnr. AT 413486, gezeigt werden, dass insbesondere die erkl- und erk2-Isoformen der MAP-Kinase funktionell durch die erfindungsgemäß verwendeten Antikörper gebunden werden können. Die Bindung der Wachstumsfaktoren an die Rezeptoren wurde dadurch verhindert bzw. reduziert. Diese Behandlung ist im Vergleich zur Immuntherapie mit Antikörpern gegen den proteinären extrazellulären Teil des EGF-Rezeptors spezifischer, da die ungewöhnlichen Tumor-assoziierten Kohlenhydratstrukturen auf EGF-Rezeptoren von normalen Zellen fehlen. Andererseits ist die Behandlung universeller, da gleichzeitig verschiedene Rezeptoren mit gleicher aberranter Glykosilierung blockiert werden.

Durch diese erfindungsgemäße Verwendung wird somit auch die mitogene Stimulierung einer Krebszelle durch EGF oder Heregulin verhindert. Die spezifische Bindung der Antikörper an eine Lewis Y Glykosilierung von Krebszellen, blockiert die Interaktion der Rezeptoren von Wachstumsfaktoren mit deren physiologischen Liganden und hemmt die Signaltransduktion durch diese Rezeptoren und damit das Zellwachstum.

Gleichzeitig kann ein solcher Antikörper durch seine Wirkung innerhalb des humoralen und zellulären Immunsystems die Tumorzelle spezifisch angreifen. Tumorzellen, die den EGF-Rezeptor bzw. Rezeptoren der EGF-Rezeptor-Familie exprimieren, werden erfindungsgemäß spezifisch gebunden und können lysiert werden.

Verfahren zur Auffindung geeigneter antigener Strukturen, Modellierung und Herstellung der von TAA abgeleiteten Peptide, Polypeptide oder Proteine bzw. dafür kodierende Nukleinsäuren, weiter Lipoproteine, Glycolipide, Kohlenhydrate oder Lipide sind dem Fachmann bekannt und können ohne unzumutbaren experimentellen Aufwand für die jeweilige Tumor-spezifische Struktur zur Verfügung gestellt werden. Weiter sind die Verfahren zur Herstellung der spezifischen Antikörper bekannt.

In einer besonderen Ausführungsform wird ein Antikörpergemisch verschiedener Antikörper mit Spezifität für TAA, insbesondere für zumindest zwei gleiche oder verschiedene Epitope eines Adhäsionsproteins, etwa eines homophilen zellulären Membranproteins, wie EpCAM verwendet. Hierbei betrifft die Erfindung eine Kombination von Antikörpern mit Spezifität für mindestens ein Lewis Y-Epitop.

Der relevante Antikörper wird erfindungsgemäß vorwiegend zur passiven Immunisierung eingesetzt, und insbesondere nur ein einziges Mal administriert. So werden etwa keine besonderen Nebenwirkungen erwartet, auch wenn der erfindungsgemäße Antikörper von einer nicht-humanen Spezies abgeleitet ist, wie etwa ein muriner Antikörper. Es wird jedoch erwartet, dass ein rekombinanter, chimärer, sowie ein mit murinen und menschlichen Bestandteilen kombinierter, humanisierter oder humaner Antikörper für die Verabreichung am Menschen besonders verträglich ist.

Unter dem Begriff "Antikörper" werden Antikörper aller Art verstanden, insbesondere monospezifische oder polyspezifische, monoklonale Antikörper, oder auch chemisch, biochemisch oder molekularbiologisch hergestellte Antikörper, oder polyklonale Antikörper mit einer bestimmten Spezifität, etwa ein Immunserum oder eine Fraktion eines Immunserums.

Der erfindungsgemäß verwendete Antikörper ist üblicherweise ein nativer Antikörper, der eventuell aus einem Organismus oder Patienten isoliert wurde. Native Antikörper sind heterotetramere Glykoproteine, die aus zwei identischen leichten Ketten und zwei identischen schweren Ketten zusammengesetzt sind.

Es kann aber auch ein Antikörper-Derivat eingesetzt werden, das bevorzugt aus der Gruppe der Antikörper-Fragmente, -Konjugate, Homologe oder Derivate, aber auch Komplexe mit zusätzlichen Effektor-Funktionen. Jedenfalls ist es bevorzugt, dass das Antikörper-Derivat zumindest Teile des Fab-Fragmentes enthält, bevorzugt gemeinsam mit zumindest Teilen des F(ab')₂ Fragmentes, und/oder Teilen der hinge Region und/oder des Fc-Teils eines lambda oder kappa Antikörpers.

Weiter kann auch ein einkettiges Antikörper-Derivat, etwa ein sogenannter "single chain" Antikörper mit Effektorfunktionen im Sinne der Erfindung herangezogen werden. Der erfindungsgemäße Antikörper ist vorzugsweise von der Art eines Immunglobulins, etwa eines IgG, IgM, IgA oder IgD.

Erfindungsgemäß bindet der Antikörper direkt an eine Tumorzelle oder an Metastasen bzw. Mikrometastasen. Ein dadurch gebildeter Immunkomplex des Antikörpers ist Voraussetzung für die humoralen und zellulären Aktivitäten des Immunsystems, ausgedrückt durch eine "antibody-dependent cellular cytotoxity" (ADCC) und/oder eine "complement dependent cytotoxicity" (CDC) Effektorfunktion. Diese Effektorfunktionen werden mit Standardtests bestimmt.

Hochaffine Antikörper sind erfindungsgemäß bevorzugt. Insbesondere werden Antikörper eingesetzt, die mit einer Affinität entsprechend einer Dissoziationskonstante unter einem Kd-Wert von 10⁻⁶ mol/l, vorzugsweise weniger als 10⁻⁷ mol/l, am meisten bevorzugt 10⁻⁸ mol/l oder weniger bindet.

Ein mögliches Behandlungsziel ist die effektive Bindung und Reduktion von Tumorzellen, um deren Disseminierung möglichst zu verhindern. Gleichzeitig werden auch insbesondere die disseminierten Tumorzellen angegriffen. Die Anzahl der in Blut, Knochenmark oder Organen detektierbaren Tumorzellen bzw. der Mikrometastasen wird erfindungsgemäß prophylaktisch und therapeutisch signifikant reduziert. Die Bildung von Metastasen soll dadurch verzögert und deren Wachstum zumindest verlangsamt werden. Durch die erfindungsgemäße Immuntherapie kann also die rückfallsfreie Lebensspanne und damit auch die Gesamtüberlebenszeit der Patienten verlängert werden. Indikator für den Behandlungserfolg ist die signifikante Reduktion von Tumorzellen in Blut, Serum oder Knochenmark.

Das erfindungsgemäß verwendete Arzneimittel liegt vorteilhafterweise in einer geeigneten Formulierung vor. Bevorzugt sind solche Formulierungen mit einem pharmazeutisch akzeptablen Träger. Dieser umfasst beispielsweise Hilfsstoffe, Puffer, Salze und Konservierungsmittel. Vorzugsweise wird eine fertige Infusionslösung zur Verfügung gestellt.

Nachdem ein Antikörper relativ stabil ist, haben Arzneimittel auf Basis von Antikörpern oder deren Derivate den wesentlichen Vorteil, dass sie als lagerstabile Lösung bzw. Formulierung in einer gebrauchsfertigen Form "ready-to-use" in den Verkehr gebracht werden können. Diese ist bevorzugt bei Kühlschranktemperaturen bis zu Raumtemperatur in der Formulierung lagerstabil. Das erfindungsgemäß verwendete Arzneimittel kann aber auch in gefrorener oder lyophilisierter Form zur Verfügung gestellt werden, die bei Bedarf aufgetaut bzw. rekonstituiert werden kann.

Die zur Verfügung gestellte Antikörperlösung kann als Bolus-Injektion oder auch in verdünnter Form intravenös verabreicht werden. Das Arzneimittel kann beispielsweise in etwa 1:10 bis 1:100 facher Verdünnung mit physiologischer Kochsalzlösung als Infusionpräparat hergestellt werden.

Zur Sättigung der relevanten Oberflächenantigene der Tumorzellen wird üblicherweise eine hohe Dosis von mindestens 50 mg, vorzugsweise mindestens 100 mg, am meisten bevorzugt mindestens 200 mg pro Patient verabreicht. Die maximale Dosis ist durch die Verträglichkeit des Antikörpers limitiert, und richtet sich nach dessen Spezifität und Avidität. Humanisierte Antikörper bzw. humane Antikörper sind am besten verträglich. Eine Dosis bis zu 1 g oder in einigen Fällen bis zu 2 g pro Patient und Behandlung kann durchaus vorteilhaft sein.

Der Patient wird üblicherweise nach dem chirurgischen Eingriff weiter untersucht, um gegebenenfalls eine geeignete Krebsbehandlung vorzunehmen. Sollte sich nach dem chirurgischen Eingriff zeigen, dass der Tumor die relevanten TAA aufweist, kann unmittelbar mit den gleichen Antikörpern, die intraoperativ eingesetzt wurden, und/oder mit immuntherapeutisch wirksamen anderen Antikörpern weiter behandelt werden.

Die übliche Behandlung zu passiven Immuntherapie umfasst mehrmalige Infusionen in regelmäßigen Abständen, etwa wöchentlich für einen Zeitraum von 6-24 Wochen, mit einer Dosis im Bereich von 1 bis 10 mg/kg, vorzugsweise im Bereich von 2 bis 6 mg/kg. Die Behandlung wird vorzugsweise in bestimmten Zeitabständen wiederholt, entsprechend der Halbwertszeit des verwendeten Antikörpers, die üblicherweise im Bereich von 5 bis 30 Tagen liegt. Durch besondere Derivatisierung des Antikörpers ist es möglich, die Halbwertszeit auf bis zu mehreren Monaten zu verlängern und dadurch die Behandlungsintervalle entsprechend zu verlängern.

Die Konzentration des Arzneimittelwirkstoffes richtet sich nach dessen Verträglichkeit. Dabei kann ein besonders verträgliches Präparat auf Basis eines humanisierten Antikörpers in hoher Konzentration direkt ohne weitere Verdünnung an den Patienten verabreicht werden. Durch die bevorzugte Konzentration im Bereich von 0,1% bis 10%, vorzugsweise von 1%-5%, ist es möglich, das verabreichte Volumen und die entsprechende Infusionszeit gering zu halten.

Die Kombination mit bekannten adjuvanten Behandlungsmethoden ist durchaus üblich. Darunter finden sich Mittel zur Radiotherapie oder Chemotherapie, wie der Monotherapie oder Polytherapie. Aus Gründen der verschiedenen Wirkmechanismen wird die Immuntherapie vorzugsweise mit der Polychemotherapie kombiniert. Bevorzugterweise für die Chemotherapie eingesetzte Mittel sind alkylierende pharmazeutische Präparationen. So sind etwa Mittel enthaltend Taxan, Anthracycline oder Platin bevorzugt. Alle üblichen Präparate, die für die verschiedenen Krebsbehandlungen zum Einsatz kommen, können erfindungsgemäß kombiniert werden. Die Chemotherapeutika werden üblicherweise intravenös oder peroral verabreicht. Perorale Verabreichungsformen der Chemotherapeutika können eventuell auch mit einer peroralen Form zur Immuntherapie erfindungsgemäß als Kombinationspräparat verabreicht werden.

Im Folgenden wird die Herstellung eines erfindungsgemäß hergestellten Arzneimittels sowie dessen intraoperative Anwendung beispielhaft beschrieben. Die folgenden Beispiele und Figuren sollen die vorliegende Erfindung weiter erläutern, aber nicht einschränken:
Figur 1 zeigt die Inhibition des Xenograft-Wachstums durch IGN311 und ABL364 nach Inokulation mit 1x10⁶ A431 Zellen in Mäusen.
Figur 2 zeigt die Inhibition des Xenograft-Wachstums durch IGN311 nach Inokulation mit 3x10⁶ A431 Zellen in Mäusen. Die Behandlung mit den Antikörpern erfolgte innerhalb eines Tages nach Dissemininierung der Tumorzellen.
Figur 3 zeigt die Tumorgewichte ohne und mit Behandlung durch IGN311 und ABL364.
Figur 4 zeigt die Reduktion der zirkulierenden EpCAM positiven Zellen im Blut nach Behandlung mit einem murinen IgG2a Mab mit EpCAM mimicking Eigenschaften.

### BEISPIELE

### Beispiel 1:

### Herstellung eines Lewis Y-Antikörper IGN311:

IGN311 ist ein humanisierter Antikörper, abgeleitet von BR55-2 murinem IgG3 Antikörper. Dieser murine Antikörper stammt von der Hybridoma-Zelllinie BR55-2 (BR55-2/IgG3), die am 17.2.1987 bei der American Type Culture Collection, Rockville MD 20852, USA unter ATCC HB 9324 hinterlegt wurde.

Der humanisierte Antikörper wurde durch CDR-grafting hergestellt (Cancer Research 56, 118-1125, March 1, 1996). Als konstante Region der schweren Kette wurde humanes IgG1 herangezogen. Die Humanisierung wurde gemäß EP 0 528 767 durchgeführt. Der Antikörper wurde in SP2/0 Zellen produziert.

Der erhaltene IGN311 zeigte sich als sehr aktiv: Humanes Komplement wurde mit einer gegenüber dem murinen Antikörper etwa 10-fach erhöhten CDC aktiviert, um Lewis Y positive Tumorzellen zu lysieren. Die Aktivität des IGN311 wurde gegen eine Auswahl von Lewis Y positiven menschlichen Tumorzelllinien in Anwesenheit von menschlichem Komplement untersucht (CDC) (SKBR5, breast cancer, SW948, colon cancer; SW2, small cell lung cancer; and MCF7, breast cancer). Die ADCC wurde ebenfalls gegen eine bestimmte Auswahl von Tumorzelllinien untersucht. Die Bindungsaffinität blieb auch nach Humanisierung etwa gleich.

Das Produkt wurde in Phosphat-gepufferter Kochsalzlösung formuliert und in einer Konzentration von 10 mg/ml abgefüllt. Diese Stammlösung wird vor Gebrauch je nach Verwendung verdünnt. Für das i.v. Produkt wird die Lösung 1: 50 mit physiologischer Kochsalzlösung verdünnt. Falls das Präparat lokal im Wundbereich appliziert wird, wird das Präparat unverdünnt eingesetzt.

### Anwendung während der Tumorresektion

Patienten mit diagnostiziertem Darmkrebs werden zur operativen Entfernung des Tumors vorbereitet. Vor der Behandlung wird eine Blutprobe entnommen. Die Behandlung mit dem i.v. Produkt erfolgt unmittelbar vor der Operation über einen Zeitraum von 2 Stunden. Die verabreichte Dosis belief sich auf 50 mg. Danach wird der Tumor innerhalb von 4 Stunden entfernt.

Blutproben werden unmittelbar nach dem Einleiten der Narkose, nach der Tumorresektion und 24 Stunden nach der Operation entnommen. Die Tumorzellen werden aus den frischen Blutproben angereichert, auf Objektträger aufgebracht und bis zur immunzytochemischen Analyse bei -20°C gelagert.

Eine Probe des entnommenen Tumorgewebes wird für die immunhistochemische Untersuchung vorbereitet.

### Tumor- und Tumorzellbestimmung

Eine immunhistochemische Bestimmung von Lewis Y Antigen wird für das operativ entfernte Tumorgewebe empfohlen. Zur histologischen Bestimmung des Lewis Y Antigens wird deparaffiniertes Gewebe mit dem murinen Antikörper BR55-2 und anschließend mit einem biotinylierten Ziegen anti-Maus IgG (Vector Laboratories) inkubiert. Unspezifische Bindungsstellen werden mit normalem Ziegenserum blockiert. Zur Bestimmung des Immunkomplexes wird Horseradish-Peroxidase-Streptavidin (1:325 in phosphatgepufferter physiologischer Kochsalzlösung) zugegeben, und eine Färbung vorgenommen. (siehe dazu J.E. Beesley; chapter 2.5.: Avidin-biotin methods in Immunocytochemistry edited by Oxford Univ. Press, 1993).

Disseminierte Tumorzellen werden aus den Blutproben folgendermaßen bestimmt.

### 1.Tumorzell-Anreicherung:

25 ml peripheres Blut wurden bei 1600 x g 20 min bei 4°C in einem OncoQuick® Röhrchen (Greiner bio-one, Altmünster, Österreich) zentrifugiert. Die Tumorzellen enthaltende Phase wurde in ein weiteres Zentrifugenröhrchen übergeführt und ein Zell-pellet durch Zentrifugieren gewonnen. Dieses Pellet wurde resuspendiert. Der zelluläre Anteil der Suspension wurde auf einen Objektträger für mikroskopische Untersuchung zentrifugiert. Der Objektträger wurde bei -20°C bis zur Auswertung gelagert.

### 2.Tumorzell-Bestimmung

Auf den Objektträger mit den isolierten und angereicherten Tumorzellen wurde eine Lösung mit fluoreszenzmarkierten spezifischen Antikörpern aufgebracht. Nach einer Inkubationszeit von 30 min wurden die durch die Antikörperbindung markierten Tumorzellen unter dem Fluoreszenzmikroskop (Axioplan Zeiss, Jena, Deutschland) sichtbar gemacht und ausgezählt. Der Gehalt an Tumorzellen in Blut wurde dann entsprechend dem Anreicherungsfaktor kalkuliert. Die Methode wurde mit Standard Tumorzell-Suspensionen validiert.

Als markierte spezifische Antikörper werden beispielsweise IGN311 (Anti-Lewis Y) und A45-B/B3 Anti-Cytokeratin: (200 µg/ml, Micromet, Martinsried, Deutschland), beide mit fluoreszierenden Proteinen konjugiert, eingesetzt.

### Beispiel 2: Hemmung des Tumorwachstums durch Antikörper gegen Lewis-Y bei Xenograft-Modellen in Nacktmäusen

Der Antikörper IGN311 (humanisierter IgG1, beispielsweise nach EP0 528 767) bzw. sein muriner Vorläufer ABL364 (IgG3, EP 0 547 079) erkennen das Lewis-Y-Antigen, einen difucosylierten Lacto-samin-Glykosidrest auf Oberflächenmolekülen. In manchen Zellen, insbesondere in Tumorzellen, werden auch Rezeptoren für Wachstumsfaktoren mit Lewis-Y-Antigen modifiziert. Dies trifft auch für die Familie der EGF-Rezeptoren (erbB1, erbB2, erbB3, erbB4, vergl. A995/2002) zu. Es soll daher mit entsprechenden humanen Tumorzelllinien (A431) untersucht werden, ob IGN311 auch in vivo in einem Xenograft-Modell das Anwachsen der Tumoren unterdrückt, das Wachstum eines bestehenden Tumors hemmt und eine disseminierte Erkrankung verhindert. Durch Vergleich des murinen Vorläufers ABL364 mit IGN311 soll auch geprüft werden, ob die Fixierung von Komplementkomponenten eine Rolle bei der Beseitigung der Tumorzellen spielt.

Ziel ist es u.a., den experimentellen Nachweis einer Wirksamkeit von IGN311 bei epithelialen Tumormodellen zu erbringen. Damit wird eine Voraussetzung für die Anwendung von IGN311 bei der Therapie von menschlichen Erkrankungen geschaffen.

### Material und Methoden:

### Material:

Das fötale Kälberserum stammt von PAA Laboratories (Linz, Austria), Dulbeccos modified Eagle Medium (DMEM, RPMI-1640) Medium, nicht-essentielle Aminosäuren, ß-Mercaptoethanol stammen von GIBCO-BRL (Grand Island, NY). Die A431 Zelllinie wurde von ATCC (Manassas, VA) erworben. L-Glutamin, Penicillin G und Streptomycin stammen von Sigma Chemical Co.

Zellkultur: A431 Zellen (humane epidermale Krebszelllinie aus Vulvakarzinom) wurden in Dulbeccos modifiziertem Eagle Medium (DMEM) enthaltend 10% Kälberserum, 4mM L-Glutamin, 100 Einheiten/ml Penicillin G und 100 µg/ml Streptomycin bei 5% CO₂ und 37°C kultiviert. SKBR3 Zellen (aus humanem Brustkrebs) wurden in RPMI-1640 Medium bei 5% CO₂ und 37°C kultiviert, wobei das Medium mit 10% Kälberserum, 2mM L-Glutamin, 100 Einheiten/ml Penicillin G und 100ug/ml Streptomycin versetzt wurde.

Tierhaltung, Xenograft-Inokulation: Pathogenfreie, 5-6 Wochen alte BALB/C nu/nu Mäuseweibchen werden für das Beispiel verwendet (Quelle: Versuchstierzucht Himberg). Das Gewicht der Tiere beträgt 18-20 g.

BALB/c (nu/nu) Mäuse müssen aufgrund ihrer Immunschwäche unter pathogenfreien Bedingungen in speziellen Filterkäfigen (Seal-Safe-IVC-Käfige, Techniplast, München) gehalten werden. Käfige mit Streu, Trinkwasser und Futter werden autoklaviert. Manipulationen an den Versuchstiere erfolgen in einer sterilen Werkbank. Die injizierten Zelllinien werden auf Kontamination mit Mykoplasmen (wofür ein PCR-Kit zur Verfügung steht) geprüft. Kulturüberstände von A431-Zellen werden stichprobenartig diesen murinen Zellkulturen zugesetzt und auf einen zytopathischen Effekt geprüft; wenn dieser auftritt, wird er als Hinweis auf das Vorhandensein einer viralen Kontamination gewertet. Naturgemäß werden diese Zellen nicht den Nacktmäusen administriert, bevor das Problem der viralen Kontamination nicht beseitigt ist.

Generieren von unterschiedlichen Tumoren in Nacktmäusen Bei jeweils 6 Mäusen pro Gruppe werden ~5*10⁶ Tumorzellen subcutan (s.c.) in den Bereich der linken Flanke injiziert: Dafür werden die A431 Zellen in 200 µl Phosphat-gepufferter Salzlösung resuspendiert; nach spätestens 6 Wochen werden die Mäuse mittels zervikaler Dislokation getötet und evaluiert.

Die Administration der Antikörper erfolgt durch intraperitoneale Injektion (2 Mal pro Woche). Die erste Administration erfolgt am nächsten Tag, d.h. etwa 21 Stunden (max. 24 Stunden) nach Applikation der Tumorzellen.

Die Tiere werden mit IGN311 über 4 Wochen behandelt. Danach werden die inneren Organe (insbesondere die Lunge) auf Ausbildung von Metastasen untersucht. Die Quantifizierung erfolgt durch Auszählen der sichtbaren Läsionen.

### Datenanalyse

Der Messparameter ist die Tumorgröße; dieses wird sowohl über das Gewicht des Tumors (= am Ende) bestimmt als aus den Dimensionen (2x wöchentlich) berechnet; letzteres erfolgt auf Grund der Messung des (größten) Längs- und Querdurchmesser und der Dicke nach der Formel für das Volumen eines Ellipsoids (12): Volumen = Länge * Breite * Höhe*π/6 bzw. bei kleinen Tumoren, deren Dicke nicht sicher bestimmt werden kann: Volumen = Länge* Breite2 * π/6. Alternativ wird das Gewicht der Tumoren festgestellt.

### Ergebnisse:

Zu Beginn wurde eine Titrationsstudie durchgeführt, um die Zahl der Tumorzellen zu bestimmen, die für die Inokulation notwendig ist. Der Bereich lag zwischen 0.5-5x10⁶ Zellen. Tumore wurden mit den A431 Zellen erhalten.

4 Mäuse/Gruppe wurden mit 1x10⁶ A431 Zellen inokuliert, die Verabreichung der Antikörper erfolgte am nächsten Tag mit einer Menge von 10mg/kg an IGN311 bzw. ABL364.

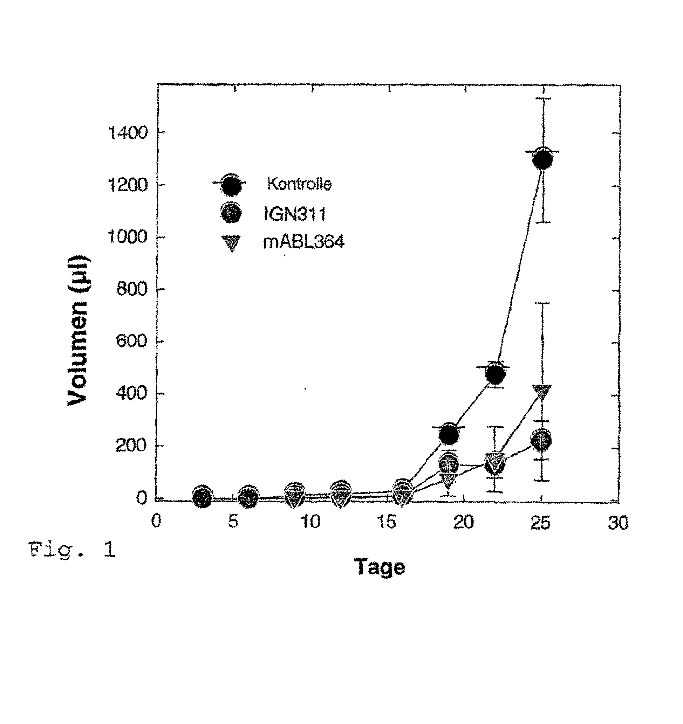

Figur 1 zeigt die Inhibition des Wachstums des Tumor-Xenografts durch IGN311 und ABL364. Die Verabreichung der Antikörper wurde innerhalb eines Tages nach der subkutanen Tumorzell-Applikation begonnen.

Am Tag 25 wurden die Tiere getötet, die Tumormasse entfernt und das Gewicht bestimmt.

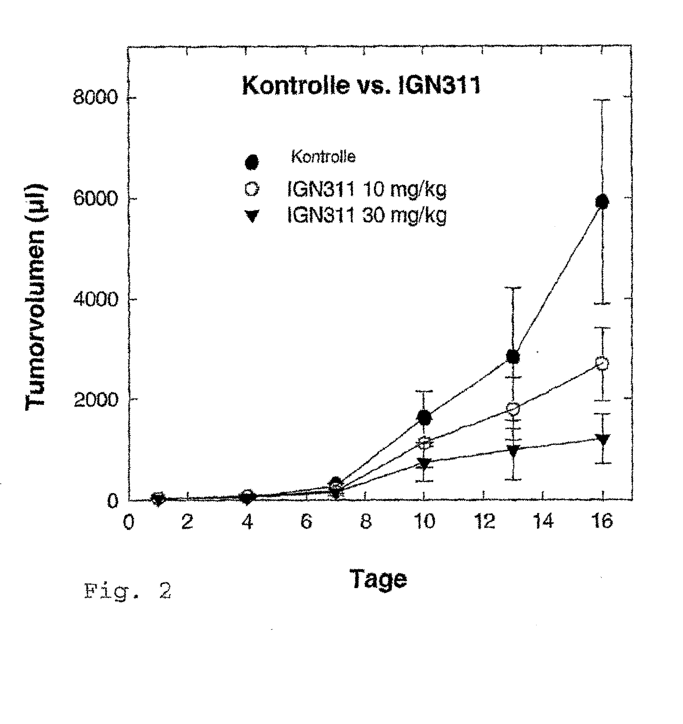

In einer weiteren Versuchsreihe wurden 3x10⁶ Tumorzellen inokuliert, was zu einer rascheren Tumorbildung führte. Hier wurde getestet, ob durch eine Erhöhung der IGN311-Dosis von 10mg/kg auf 30mg/kg zu einer zusätzlichen Wachstumshemmung führte. Wie aus Fig. 2 ersichtlich ist, zeigt IGN311 auch bei erhöhter Dosis eine inhibitorische Wirkung.

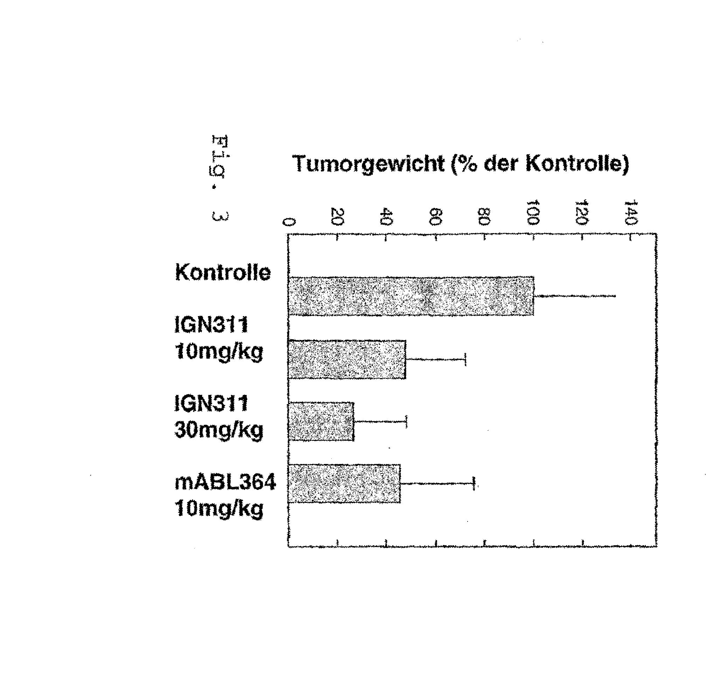

Die Bestimmung des Tumorvolumens erwies sich als schwierig, da die Tumoren nicht als ellipsoide Körper wuchsen. Daher wurden die Tiere am 16. Tag getötet und das Gewicht der Tumormasse bestimmt. Die Ergebnisse sind Figur 3 zu entnehmen.

Basierend auf den erhaltenen Daten kann festgestellt werden, dass in diesem Xenograft Modell IGN311 eine signifikante Antitumor-Aktivität in vivo zeigt und der Effekt des IGN311 nicht dramatisch durch eine Erhöhung der Dosis von 10mg/kg auf 30mg/kg gesteigert wird.

In einem alternativen Versuchsansatz erfolgt der Nachweis der Hemmung des Tumorwachstums bzw. der Disseminierung der Tumorzellen durch den Antikörper dadurch, dass die Antikörper innerhalb von maximal 24 Stunden vor der Applikation der Tumorzellen subkutan oder intravenös verabreicht werden. Die Tumorzellen können wieder subkutan, intravenös oder direkt in die Lunge der Versuchstiere appliziert werden.

Das Tumorwachstum wird an den lebenden Tieren über die Messung der Tumorgröße bestimmt, nach mehreren Tagen bzw. Wochen werden die Tiere durch zervikale Dislokation getötet und die Tumorgröße bestimmt. Weiters werden die Organe entnommen und mittels verschiedener Verfahren auf Mikrometastasen und andere tumorbedingte Veränderungen untersucht.

### Beispiel 3: Nachweis der Reduktion zirkulierender EpCAM Zellen im Blut

### Patienten, Material und Methoden:

Zur Vakzinierung wurde ein muriner IgG2a Mab, mit EpCAM Mimick-Eigenschaften verwendet. Der Antikörper wurde in einer Menge von 0,5mg Mab adsorbiert auf 1,67 mg Aluminium Hydroxid in 0,5 ml Puffer subkutan verabreicht.

Als Probanden wurden an Krebs erkrankte Patienten (älter als 19 Jahre) ausgewählt, bei denen durch konventionelle Therapie keine Erfolge erzielt werden konnten. Die Behandlung erfolgte mit 0,5 mg Antikörper, subkutan an den Tagen 1, 15, 29, 57.

### Analyse von zirkulierenden Tumorzellen in peripherem Blut:

5 ml heparinisiertes Blut wurde an den Tagen 1, 29 und 71 entnommen. Nach milder Erithrozyten-Lyse wurde die Restflüssigkeit mit para-magnetischem anti-EpCAM Antikörper inkubiert und die Zellen auf einer magnetischen Säule aufgetrennt (Miltenyi). Nach der Elution wurden die gebundenen Zellen mit FITC-anti-EpCAM Antikörper markiert und analysiert bzw. in einem Inversions-Fluoreszenz-Mikroskop gezählt.

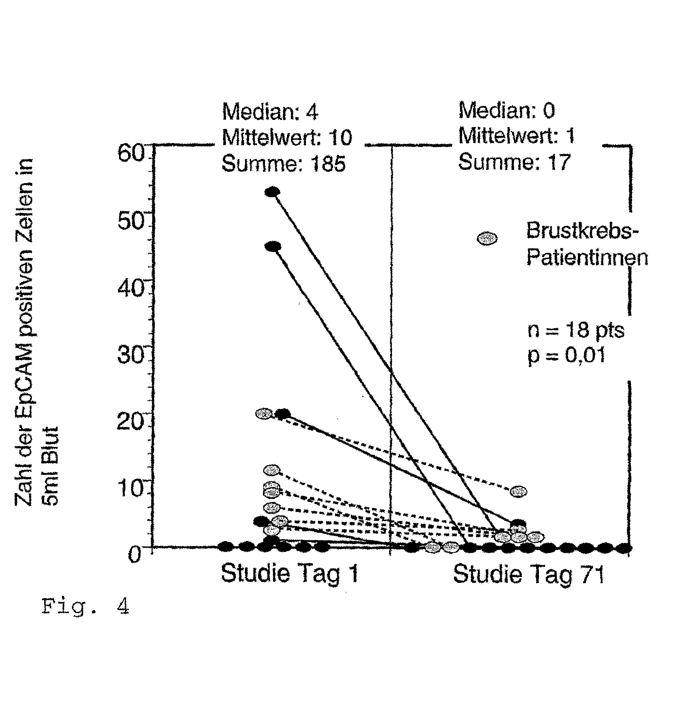

Die Ergebnisse sind der Figur 4 zu entnehmen, worin die Reduktion der zirkulierenden EpCAM Zellen im Blut deutlich zu sehen ist.

### Beispiel 4: Verabreichung des IGN311 an Patienten mit Pleuralerguss

In einer klinischen Untersuchung wird Patienten (18 bis 80 Jahre), die an Krebs erkrankt sind und einen Pleuralerguss aufweisen, 100 mg IGN311 in einer Einmalgabe intravenös maximal 24 Stunden vor Entfernung des Ergusses verabreicht.

Sowohl das Volumen der Pleuraflüssigkeit, die durch die Drainage abgeleitet wird, als auch das Vorhandensein von Lewis y positiven Tumorzellen werden bestimmt. Die biologische Aktivität des IGN311 in der pleuralen Effusion kann durch CDC Bestimmung erfolgen. Weiters kann bestimmt werden, ob die Zeit, bis die Pleurodese durchgeführt werden kann, das bedeutet bis die Pleuraflüssigkeit abgeronnen ist, durch die Gabe des Antikörpers verkürzt werden kann.

## Patentansprüche

1. Verwendung eines Präparates auf Basis eines Antikörpers gerichtet gegen ein Lewis Y zur Herstellung eines Arzneimittels zur intraoperativen Behandlung von Tumorpatienten durch Immunkomplexierung von Tumorzellen im Rahmen von chirurgischen Eingriffen, wobei das Präparat zur prophylaktischen Behandlung zur Verhinderung der Disseminierung von Tumorzellen eingesetzt wird, und wobei die Tumorzelle eine epitheliale Tumorzelle ist, und wobei das Arzneimittel unmittelbar während oder innerhalb von 24 Stunden vor dem chirurgischen Eingriff verabreicht wird.

2. Verwendung nach Anspruch 1, **dadurch gekennzeichnet, dass** der Antikörper in einem Antikörpergemisch von verschiedenen Antikörpern mit Spezifität für Tumor-assoziierte Antigene verwendet wird.

3. Verwendung nach Anspruch 1 oder 2, **dadurch gekennzeichnet, dass** der Antikörper das Immunsystem funktionell aktiviert, entsprechend einer ADCC und CDC-Effektorfunktion.

4. Verwendung nach einem der Ansprüche 1 bis 3, **dadurch gekennzeichnet, dass** der Antikörper an Lewis Y mit einer Affinität entsprechend einer Dissoziationskonstante unter einem Kd-Wert von 10⁻⁶ mol/l, vorzugsweise weniger als 10⁻⁷mol/l, am meisten bevorzugt 10⁻⁸ mol/l oder weniger bindet.

5. Verwendung nach einem der Ansprüche 1 bis 4, **dadurch gekennzeichnet, dass** der Antikörper von murinen, chimären, humanisierten und/oder humanen Quellen abgeleitet ist.

6. Verwendung nach einem der Ansprüche 1 bis 5, **dadurch gekennzeichnet, dass** das Arzneimittel systemisch mit einer einmaligen Dosis von mindestens 50 mg, vorzugsweise mindestens 100 mg, am meisten bevorzugt mindestens 200 mg, bis zu 2 g pro Patient, verwendet wird.

7. Verwendung nach einem der Ansprüche 1 bis 6, **dadurch gekennzeichnet, dass** das Arzneimittel lokal zum Tumorgewebe und/oder zum Wundbereich appliziert wird.

8. Verwendung nach einem der Ansprüche 1 bis 7, **dadurch gekennzeichnet, dass** das Arzneimittel innerhalb von 4 Stunden vor dem chirurgischen Eingriff verabreicht wird.

9. Verwendung nach einem der Ansprüche 1 bis 8, **dadurch gekennzeichnet, dass** der chirurgische Eingriff zur Biopsie und/ oder Entfernung eines soliden Tumors vorgenommen wird.

10. Verwendung nach einem der Ansprüche 1 bis 9, **dadurch gekennzeichnet, dass** der chirurgische Eingriff zur Bestimmung über die Malignität eines Tumors vorgenommen wird.

11. Verwendung nach einem der Ansprüche 1 bis 10, **dadurch gekennzeichnet, dass** der Antikörper nach dem chirurgischen Eingriff am immunkomplexierten Tumorgewebe bestimmt wird.

12. Verwendung nach einem der Ansprüche 1 bis 11, **dadurch gekennzeichnet, dass** der Antikörper an Tumorzellen in Blut- oder Serumproben bestimmt wird.

13. Präparat auf Basis eines Antikörpers gerichtet gegen ein Lewis Y zur Verwendung in einer intraoperativen Behandlung von Tumorpatienten durch Immunkomplexierung von Tumorzellen im Rahmen von chirurgischen Eingriffen, wobei das Präparat zur prophylaktischen Behandlung zur Verhinderung der Disseminierung von Tumorzellen eingesetzt wird, und wobei die Tumorzelle eine epitheliale Tumorzelle ist, und wobei das Arzneimittel unmittelbar während oder innerhalb von 24 Stunden vor dem chirurgischen Eingriff verabreicht wird.

14. Präparat zur Verwendung nach Anspruch 13, wobei die Verwendung weiters wie in einem der Ansprüche 2 bis 12 definiert ist.

## Claims

1. Use of a preparation based on an antibody directed against a Lewis Y for producing a medicament for the intraoperative treatment of tumor patients through immunocomplexing of tumor cells in the context of surgical interventions, wherein the preparation is used for prophylactic treatment to prevent the dissemination of tumor cells, and wherein the tumor cell is an epithelial tumor cell, and wherein the medicament is administered directly during or within 24 hours prior to the surgical intervention.

2. Use according to claim 1 **characterized in that** the antibody is used in an antibody mix of various antibodies with specificity for tumor-associated antigens.

3. Use according to claim 1 or 2 **characterized in that** the antibody functionally activates the immune system in accordance with an ADCC and CDC effector function.

4. Use according to any one of claims 1 to 3 **characterized in that** the antibody binds to Lewis Y with an affinity corresponding to a dissociation constant below a Kd value of 10⁻⁶ mol/l, preferably less than 10⁻⁷ mol/l and most preferably 10⁻⁸ mol/l or less.

5. Use according to any one of claims 1 to 4 **characterized in that** the antibody is derived from murine, chimeric, humanized and/or human sources.

6. Use according to any one of claims 1 to 5 **characterized in that** the medicament is used systemically with single dose from at least 50 mg, preferably at least 100 mg, most preferably 200 mg, up to 2 g per patient.

7. Use according to any one of claims 1 to 6 **characterized in that** the medicament is applied locally to the tumor tissue and/or the wound area.

8. Use according to any one of claims 1 to 7 **characterized in that** the medicament is administered within 4 hours prior to the surgical intervention.

9. Use according to any one of claims 1 to 8 **characterized in that** the surgical intervention is carried out for the biopsy and/or removal of a solid tumor.

10. Use according to any one of claims 1 to 9 **characterized in that** the surgical intervention is carried out to determine the malignancy of the tumor.

11. Use according to any one of claims 1 to 10 **characterized in that** after the surgical intervention the antibody is determined on immunocomplexed tumor tissue.

12. Use according to any one of claims 1 to 11 **characterized in that** the antibody is determined on tumor cells in blood and serum samples.

13. Product based on an antibody directed against a Lewis Y for producing a medicament for the intraoperative treatment of tumor patients through immunocomplexing of tumor cells within the scope of surgical interventions, wherein the preparation is used for prophylactic treatment to prevent the dissemination of tumor cells, and wherein the tumor cell is an epithelial tumor cell, and wherein the medicament is administered directly during or within 24 hours prior to the surgical intervention.

14. Preparation for use according to claim 13 wherein the use is further defined in any one of claims 2 to 12.

## Revendications

1. Utilisation d'une préparation à base d'un anticorps dirigé contre un antigène Lewis Y pour la production d'un médicament pour le traitement intra-opératoire de patients présentant une tumeur, par formation d'un complexe immun de cellules tumorales dans le cadre d'interventions chirurgicales, dans laquelle la préparation est utilisée pour le traitement prophylactique pour empêcher la dissémination de cellules tumorales et dans laquelle la cellule tumorale est une cellule épithéliale tumorale et dans laquelle le médicament est administré immédiatement pendant l'intervention chirurgicale ou dans les 24 heures précédant l'intervention chirurgicale.

2. Utilisation selon la revendication 1, **caractérisée en ce que** l'anticorps est utilisé dans un mélange d'anticorps de différents anticorps présentant une spécificité pour des antigènes associés à une tumeur.

3. Utilisation selon la revendication 1 ou 2, **caractérisée en ce que** l'anticorps active fonctionnellement le système immunitaire, de façon correspondante à une fonction effectrice ADCC et CDC.

4. Utilisation selon l'une des revendications 1 à 3, **caractérisée en ce que** l'anticorps se lie à l'antigène Lewis Y avec une affinité correspondant à une constante de dissociation inférieure à une valeur Kd de 10⁻⁶ moles/l, de préférence, inférieure à 10⁻⁷ moles/l, de manière préférée entre toutes, de 10⁻⁸ moles/l ou inférieure.

5. Utilisation selon l'une des revendications 1 à 4, **caractérisée en ce que** l'anticorps est dérivé de sources murines, chimériques, humanisées et/ou humaines.

6. Utilisation selon l'une des revendications 1 à 5, **caractérisée en ce que** le médicament est utilisé de façon systémique en une dose unique d'au moins 50 mg, de préférence, d'au moins 100 mg, de manière préférée entre toutes, d'au moins 200 mg, jusqu'à 2 g par patient.

7. Utilisation selon l'une des revendications 1 à 6, **caractérisée en ce que** le médicament est appliqué localement sur le tissu tumoral et/ou dans la zone de la plaie.

8. Utilisation selon l'une des revendications 1 à 7, **caractérisée en ce que** le médicament est administré dans les 4 heures précédant l'intervention chirurgicale.

9. Utilisation selon l'une des revendications 1 à 8, **caractérisée en ce que** l'intervention chirurgicale est entreprise pour effectuer la biopsie et/ou l'excision d'une tumeur solide.

10. Utilisation selon l'une des revendications 1 à 9, **caractérisée en ce que** l'intervention chirurgicale est entreprise pour déterminer la malignité d'une tumeur.

11. Utilisation selon l'une des revendications 1 à 10, **caractérisée en ce que** l'anticorps est déterminé après l'intervention chirurgicale sur le tissu tumoral immuno-complexé.

12. Utilisation selon l'une des revendications 1 à 11, **caractérisée en ce que** l'anticorps est déterminé sur des cellules tumorales dans des échantillons de sang ou de sérum.

13. Préparation à base d'un anticorps dirigé contre un antigène Lewis Y pour son utilisation dans un traitement intra-opératoire de patients présentant une tumeur, par formation d'un complexe immun de cellules tumorales dans le cadre d'interventions chirurgicales, dans laquelle la préparation est utilisée pour le traitement prophylactique pour empêcher la dissémination de cellules tumorales et dans laquelle la cellule tumorale est une cellule tumorale épithéliale et dans laquelle le médicament est administré immédiatement pendant l'intervention chirurgicale ou dans les 24 heures précédant l'intervention chirurgicale.

14. Préparation pour son utilisation selon la revendication 13, dans laquelle l'utilisation est définie en outre comme dans l'une des revendications 2 à 12.
